# NEUE EUROPÄISCHE PATENTSCHRIFT

(11) **EP 3 528 748 B2**
(45) Veröffentlichungstag und Bekanntmachung des Hinweises auf die Entscheidung über den Einspruch: **14.02.2024**
(45) Hinweis auf die Patenterteilung: 20.01.2021
(21) Anmeldenummer: 17788212.3
(22) Anmeldetag: 24.10.2017
(51) Int. Cl.: A61F 2/07, A61F 2/24, A61F 2/90

(54) **INTRALUMINALE GEFAESSPROTHESE ZUR IMPLANTATION IN DAS HERZ ODER HERZGEFAESSE EINES PATIENTEN**
INTRALUMINAL VESSEL PROSTHESIS FOR IMPLANTATION INTO THE HEART OR CARDIAC VESSELS OF A PATIENT
PROTHÈSE VASCULAIRE INTRALUMINALE DESTINÉE À ÊTRE IMPLANTÉE DANS LE C UR OU DANS DES VAISSEAUX CARDIAQUES D'UN PATIENT

(30) Priorität: 24.10.2016 DE 202016105963 U
(43) Veröffentlichungstag der Anmeldung: 28.08.2019
(73) Patentinhaber: NVT AG, 5630 Muri AG (CH)
(72) Erfinder: KAWA, Emilia, 72401 Haigerloch (DE); CENTOLA, Marcos, CEP 15025010 S.J.Do Rio Preto São Paulo (BR); KUETTING, Maximilian, 71032 Boeblingen (DE)
(74) Vertreter: Witte, Weller & Partner Patentanwälte mbB
(86) Internationale Anmeldenummer: PCT/EP2017/077044
(87) Internationale Veröffentlichungsnummer: WO 2018/077821

(56) Entgegenhaltungen:
- EP-A1- 2 926 767
- EP-A2- 0 657 147
- WO-A1-2010/006627
- WO-A1-2011/051043
- WO-A1-2012/032187
- WO-A2-01/49213
- WO-A2-2009/094188
- DE-A1- 10 301 026
- US-A1- 2003 040 792
- US-A1- 2003 236 568
- US-A1- 2004 082 989
- US-A1- 2004 236 411
- US-A1- 2005 096 734
- US-A1- 2005 222 674
- US-A1- 2008 036 113
- US-A1- 2008 177 381
- US-A1- 2011 125 249
- US-A1- 2013 090 714
- US-A1- 2014 039 614
- US-A1- 2015 164 662
- US-B1- 6 579 314
- US-B2- 9 693 860

## Beschreibung

Die vorliegende Erfindung betrifft eine intraluminale Gefäßprothese zur Implantation in das Herz und/oder in Herzgefäße eines Patienten, insbesondere zur Verankerung im Bereich der Herzklappensegel; die Gefäßprothese weist ein distales und ein proximales Ende auf, sowie ein Stent-Gerüst und ein Prothesen-Material, mit welchem das Stent-Gerüst zumindest teilweise bedeckt ist, so dass ein gecoverter proximaler Abschnitt und ein nicht-gecoverter distaler Abschnitt gebildet wird.

Allgemein werden intraluminale Gefäßprothesen im Stand der Technik insbesondere zur Behandlung von Aneurysmen bzw. zum Ersatz oder zur Unterstützung der natürlichen Gefäße oder sogar Herzklappen eingesetzt.

Unter einem Aneursyma versteht man dabei eine Arterienerweiterung oder arterielle Aussackung, bei der sich infolge angeborener oder erworbener Wandveränderungen der Querschnitt von Blutgefäßen spindel- oder sackförmig lokal erweitert. Gefäßprothesen, die zur Behandlung von Aneurysmen eingesetzt werden. Dabei unterscheidet man zwischen einem echten Aneurysma, einem falschen Aneurysma und einem dissezierenden Aneurysma (*Aneurysma dissecans*), das als Folge einer Gefäßwanddissektion auftritt. Unter letzterer ist eine Aufspaltung der Wandschichten der Aorta zu verstehen, die meist durch einen Einriss der inneren Gefäßwand verursacht ist, mit nachfolgender Einblutung zwischen den Schichten. Zu den Risikofaktoren, die zu einer Aortendissektion führen können, zählen eine Strukturschwäche der Gefäßwand sowie die Arteriosklerose.

Eine Aortendissektion ist in aller Regel unmittelbar lebensbedrohlich, weil sie zu einer Aortenruptur und zu akuten Durchblutungsstörungen verschiedener Organe führen kann. Eine unverzügliche Diagnostik ist deshalb bei dieser Krankheit von entscheidender Bedeutung.

Statistisch gesehen treten Aortendissektionen am häufigsten (zu ca. 65%) nur wenige Zentimeter oberhalb der Aortenklappe, im ansteigenden Abschnitt der Aorta (*Aorta ascendens*) auf. Am zweithäufigsten (mit ca. 20%) entstehen Aortendissektionen unmittelbar nach Abgang der linken Schlüsselbeinarterie (*Arteria subclavia sinistra*) im absteigenden Abschnitt der Aorta (*Aorta descendens*). Des Weiteren sind der Aortenbogen mit 10 % oder die Bauchschlagader (*Aorta abdominalis*) mit 5 % betroffen.

Je nach lokalem Auftreten einer Dissektion, wird diese in "A" oder "B" klassifiziert, wobei sich eine strikte Unterscheidung zwischen Aortendissektionen mit und ohne Beteiligung der *Aorta ascendens* durchgesetzt hat. Dabei werden in der Regel Dissektionen, bei welchen der Eintritt im Bereich der *Aorta ascendens* liegt, als Typ A-Dissektionen bezeichnet, und Dissektionen, bei welchen der Eintritt ("Entry") distal der linken *Arteria subclavia* liegt als Typ-B-Dissektionen. Wie bereits erwähnt, ist die Aortendissektion ist einer der dringlichsten Notfälle in der Kardiologie und Herzchirurgie. Die hohe Sterblichkeit von ein bis zwei Prozent pro Stunde im Fall der Typ-A-Dissektion in der Akutphase zwingt zu einer unverzüglichen Klärung der Verdachtsdiagnose.

Bei einer akuten Dissektion Typ A ist es vor diesem Hintergrund äußerst wichtig, die Gefahr einer Aortenruptur rasch zu bannen, wobei gegenwärtig üblicherweise künstliche Gefäßprothesen zum sofortigen operativen Ersatz der *Aorta ascendens* eingesetzt werden. Bei Notfalleinsätzen seltener sind hingegen Rekonstruktionen der Aortenklappe. In der Regel wird daher die Aortenklappe bei einer Dissektion der klappennahen Aortenabschnitte und bei Patienten mit einer angeborenen Bindegewebserkrankung entfernt und eine Prothese mit integrierter Klappenprothese eingesetzt, an die auch die Herzkranzgefäße wieder "angeschlossen" werden. Grundsätzlich werden dabei zwei Arten von Herzklappenprothesen unterschieden, mechanische Klappen einerseits, die künstlich hergestellt werden und zum größten Teil aus Metall bestehen, sowie biologische Klappen andererseits, die als Transplantate von Mensch oder Tier zur Verfügung stehen.

Die künstlichen Prothesen können dabei entweder offen-chirurgisch oder minimal-invasiv eingesetzt werden.

Die korrekte Einsetzung der Prothese an die zu behandelnde Stelle stellt regelmäßig eine Herausforderung an den behandelnden Arzt dar, da bereits ein geringfügiges Verrutschen der Prothese die "stillgelegte" Aussackung wieder in den Blutkreislauf einbeziehen kann, und dadurch die Gefahr einer Ruptur erneut gegeben ist. Die Gefahr des Verrutschens bzw. einer Migration der Prothese ist auch nach deren Platzierung gegeben, insbesondere vor dem Hintergrund der natürlichen Herzbewegung. Aus diesen Gründen wird die Prothese in der Regel proximal und distal durch Nähte an der Gefäßwand fixiert. Diese Naht-Fixierung hat allerdings den Nachteil, dass die oftmals ohnehin beeinträchtigten Gefäßwände weiter strapaziert werden. Auch ist bei Patienten mit stark beschädigter Gefäßwand ein Fixieren mittels Nähten nicht möglich.

Die WO 01/49213 offenbart eine intraluminale Gefäßprothese zur Implantation in das Herz eines Patienten. Das Stent-Gerüst der intraluminalen Gefäßprothese weist einen proximalen und distalen Verankerungsabschnitt auf, wobei die Gefäßprothese insgesamt gecovert ist.

Auch die WO 2009/094188 A2 offenbart eine vollständig gecoverte Gefäßprothese mit Verankerungsstrukturen am proximalen Ende.

Vor diesem Hintergrund ist Aufgabe der vorliegenden Erfindung, eine Vorrichtung bereitzustellen, mit welcher die beschriebenen Nachteile überwunden und Herzklappenprothesen sicher im Herzen, insbesondere bei Typ-A-Dissektionen, fixiert werden können.

Erfindungsgemäß wird dies durch eine Gefäßprothese der eingangs genannten Art gelöst, wobei die Gefäßprothese am proximalen Ende zumindest eine bis drei ängliche drahtförmige Verankerungsstruktur(en) aufweist, welche im Wesentlichen schlaufenförmig ist/sind, und welche jeweils mit zwei Enden am Stent-Gerüst fixiert ist/sind und welche jeweils mit ihrem proximalen Ende in Form einer Schlaufe in proximale Richtung weisend und über das proximale Ende des Stent-Gerüsts hinaus ragend positioniert ist/sind.

Die der Erfindung zugrunde liegende Aufgabe wird auf dieser Weise vollkommen gelöst.

Mit der am proximalen Ende vorgesehenen spezifischen Verankerungsstruktur der Gefäßprothese wird erreicht, dass diese sicher im Herzen verankert wird, ohne die natürliche Funktion der Herzklappen zu stören. Dies wird durch die spezifische Ausgestaltung der Verankerungsstruktur erreicht, die sich am proximalen Ende schlaufenartig bzw. bogenartig in proximale Richtung erstreckt. Über dieser Verankerungsstruktur kann die Gefäßprothese gezielt und sicher positioniert werden.

Die erfindungsgemäße Gefäßprothese dient dabei dann als Anker für eine separat einzubringende Herzklappenprothese, die zumindest teilweise oder überschneidend in der im Gefäß verankerten erfindungsgemäßen Gefäßprothese freigesetzt wird, und die der Unterstützung oder dem Ersatz der natürlichen Gefäßprothese dient.

Dabei kann jede Herzklappenprothese über die erfindungsgemäße Gefäßprothese eingesetzt werden, welche Herzklappenprothese vorzugsweise ein selbstexpandierendes Gerüst mit darin angebrachten Klappen aufweist. Beispielhaft wird auf die EP 2 724 690 verwiesen, in der Beispiele für Herzklappen offenbart sind. Dem Fachmann wird anhand der vorliegenden offenbar sein, welche Herzklappen zum Einsatz mit der erfindungsgemäßen Gefäßprothese geeignet sind.

Mit der Verankerungsstruktur wird erreicht, dass ein zusätzliches Vernähen der Gefäßprothese bzw. der Herzklappenprothese an den Gefäßen und damit eine Belastung dieser vermieden werden kann. Die erfindungsgemäße Gefäßprothese stellt damit eine Art Ankerstruktur bzw. Aufnahmestruktur dar, über welchen die in einem zweiten Schritt einzuführende Herzklappenprothese sicher im Herzen verankert werden kann. Dadurch wird auch vermieden, dass an der Herzklappenprothese selber komplizierte Fixierungselemente vorgesehen sein müssen, um diese sicher im Herzen zu fixieren.

Die Verankerungsstruktur wird dabei vorzugsweise hinter einem Segel der Aortenklappe, vorzugsweise das nonkoronare Segel bzw. "non coronary cusp "NCC", positioniert, während die Gefäßprothese selber mit ihrem restlichen Körper noch im Katheter über eine Rückzugshülle oder eine andere Komprimierung fixiert ist. Dadurch kann die Verankerungsstruktur genau ausgerichtet werden und eine spätere proximale Migration der Prothese verhindern.

Die erfindungsgemäße Gefäßprothese dient dabei vorzugsweise der Behandlung von Aortendissektionen vom Typus A, und wird über eine kathetergeführte Anwendung minimal-invasiv eingebracht.

Grundsätzlich werden bei Gefäßprothesen bzw. endoluminalen Stentgrafts allgemein sowie vorliegend zur Bezeichnung der jeweiligen Enden der Gefäßprothese die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen, mit welchen die Gefäßprothesen in die Gefäße eingebracht werden, bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt.

Erfindungsgemäß bedeutet die Einteilung des Grundköpers der Gefäßprothese in einen proximalen Abschnitt und einen distalen Abschnitt, dass sich die jeweiligen Abschnitte durch eine voneinander unterschiedlichen Bauweise unterscheiden, und bspw. eine unterschiedliche Anzahl an Stent-Ringen oder unterschiedliche Länge des Stent-Gerüsts aufweisen, bzw. deren Stent-Ringe/Stent-Gerüst unterschiedliche Durchmesser aufweisen, und/oder von Prothesen-Material bedeckt ("gecovert") oder unbedeckt ("un-gecovert/nicht-gecovert") sind. Das Prothesen-Material kann dabei bspw. angenäht oder aufgeschrumpft oder anderweitig an dem Stent-Gerüst angebracht sein, und zwar entweder auf der der Gefäßwand zugewandten Seite des Stent-Gerüsts, oder auf der Innenseite, dem Blutfluss zugewandten Seite.

Der von Prothesenmaterial bedeckte, "gecoverte" proximale Abschnitt soll dabei die Dissektion überbrücken, der un-gecoverte distale Abschnitt wird distal der Dissektion gesetzt und dient überwiegend - wie auch der gecoverte Abschnitt - der Fixierung der Gefäßprothese im Gefäß.

Eine "drahtförmige, im Wesentlichen schlaufenförmige" Verankerungsstruktur bedeutet vorliegend, dass die Struktur durch Biegen eines Drahtes oder drahtähnlicher Struktur in einen Bogen gebildet wird, mit zwei "freien" Enden sowie einem Bogen-Ende, der den beiden freien Enden gegenüber liegt. "Freie Enden" bedeutet dabei, dass diese nicht - wie der Bogenabschnitt - direkt bzw. in einem Stück miteinander verbunden sind.

"Im Wesentlichen" bedeutet ferner, dass die Verankerungsstruktur nicht exakt schlaufenförmig-rund gebogen sein muss, sondern lediglich nach Art einer Schlaufe geführt sein soll und von einem Fachmann noch als solche betrachtet wird.

Erfindungsgemäß ragt die Verankerungsstruktur über das proximale Ende der Gefäßprothese in proximale Richtung hinaus, wobei sich die Verankerungsstruktur mit zwischen ca. 1 cm und 3 cm, vorzugsweise mit ca. 1, 1,5, 2, 2,5 oder 3 cm über das proximale Ende der Gefäßprothese hinaus erstreckt.

Das Prothesenmaterial kann ein Material aufweisen oder aus diesem gebildet sein, das ausgewählt ist aus Polyester, Polyurethan, Polystyrol, Polytetrafluorethylen, ultrahochmolekulargewichtiges Polyethylen (UHMPE), oder Mischungen davon.

Unter einem "Stent-Gerüst" wird vorliegend jede Drahtprothese in Röhrchenform bzw. Zylinderform aus Metall oder Kunstfaser verstanden, das vorzugsweise selbstexpandierend ist, und ein gitter- oder netzartiges Gerüst bildet, an welches ggf. Prothesenmaterial angebracht werden kann. Hierunter kann bspw. ein Drahtgeflecht zu verstehen sein, oder aber hintereinander angeordnete, mäanderförmig umlaufende, sogenannte Stent-Federn/Stent-Ringe, die ggf. über Verbindungsstützen aus Draht miteinander verbunden sind, oder die lediglich über das Implantatmaterial miteinander verbunden sind. Das Stent- Gerüst ist dabei üblicherweise aus einem Shape-Memory-Material, in der Regel aus Nitinol, wodurch das Gerüst nach Einbringung in ein Gefäß zur ²Freisetzung wieder in den expandierten Zustand übergehen und das Gefäßimplantat dadurch "aufspannen" kann.

Entsprechend ist gemäß einer Ausführungsform der vorliegenden Erfindung das Stent-Gerüst ein laser-geschnittenes Stent-Gerüst oder ein geflochtenes oder gewebtes Stent-Gerüst, oder besteht, ggf. nur teilweise, (bspw. im proximalen Abschnitt oder im distalen Abschnitt) aus einzelnen, nur über das Prothesenmaterial und nicht untereinander (durch Stege o.ä.) verbundenen Stentringen.

Dem Fachmann wird dabei unter Berücksichtigung der jeweils zu behandelnden Dissektion und des Gefäßzustandes klar und offensichtlich sein, welches Stent-Gerüst eingesetzt wird, um die bestmöglichste Verwirklichung der Erfindung zu erreichen. Entsprechend kann der proximale Abschnitt eine andere Stent-Gerüst-Struktur aufweisen als der distale Abschnitt der Gefäßprothese, oder aber beide weisen das gleiche Stent-Gerüst auf.

Entsprechend weist in einer bevorzugten Ausführungsform das Stent-Gerüst rautenförmige Zellen auf bzw. besteht aus umlaufenden Reihen hintereinander angeordneter rautenförmiger Zellen, die über ihre Ecken aneinander angrenzen bzw. miteinander verbunden sind. An den äußersten Enden der Gefäßprothese befinden sich proximal und distal entsprechend freie "Ecken" der rautenförmigen Zellen.

Gemäß einer Ausführungsform der erfindungsgemäßen Gefäßprothese sind am distalen Ende der Gefäßprothese Fixierstrukturen vorgesehen, über welche das distale Ende der Gefäßprothese an einem Freisetzungskatheter fixiert und komprimiert gehalten werden kann. Vorzugsweise sind die Fixierstrukturen T-förmige Verlängerungen von zumindest drei freien Ecken der rautenförmigen Zellen am distalen Ende. Diese T-förmigen Strukturen können in entsprechende T-förmige Aufnahmen in dem Freisetzungskatheter eingreifen. Eine Rückzugshülle, die dann über das distale Ende der Gefäßprothese geführt wird, hält die T-förmigen Fixierstrukturen in den T-förmigen Aufnahmen und fixiert damit das distale Ende der Gefäßprothese an dem Freisetzungskatheter. Bei einem Zurückziehen der die Gefäßprothese komprimierenden Rückzugshülle über die T-förmigen Aufnahme hinweg, können sich die T-förmigen Fixierstrukturen aus den Aufnahmen lösen, wodurch das distale Ende der Gefäßprothese freigesetzt wird.

In einer Ausführungsform der erfindungsgemäßen Gefäßprothese ist bevorzugt, wenn die distalen Enden der drahtförmigen Verankerungsstruktur an unmittelbar benachbarten Ecken zweier rautenförmigen Zellen des proximalen Endes der Gefäßprothese positioniert sind.

Mit dieser Ausführungsform wird erreicht, dass die Verankerungsstruktur stabil "aufgespannt" ist, und ein Vergrößern der Schlaufe, was ggf. eine Migration der Gefäßprothese zur Folge hätte, vermieden wird. Durch die vergleichsweise enge Fixierung der distalen Enden der Schlaufe wird über die proximale Schlaufe der Verankerungsstruktur dieser eine Steifigkeit verliehen, was wiederum eine stabile Verankerung der Gefäßprothese sichert.

Erfindungsgemäß ist vorgesehen, dass die drahtförmige Verankerungsstruktur an/in ihrem proximalen Ende bzw. Endbereich eine trapezförmige Erweiterung umfasst.

Durch die trapezförmige Erweiterung erhält die Verankerungsstruktur eine zusätzliche Stabilität in diesem Bereich, mit welcher die sichere Verankerung in der Gefäßwand zusätzlich gesichert ist.

Dabei ist gemäß einer Weiterbildung bevorzugt, wenn das äußerste proximale Ende der drahtförmigen Verankerungsstruktur tropfenförmig ist.

Diese Ausführungsform führt zu einer noch verbesserten Stabilität der Verankerungsstruktur. "Tropfenförmig" bedeutet dabei eine in proximale Richtung weisende zusätzlich zur Basis-Schlaufenform kleine aufgesetzte Schlaufe.

Bei der erfindungsgemäßen intraluminalen Gefäßprothese ist gemäß einer Ausführungsform bevorzugt, wenn das Stent-Gerüst und die Verankerungsstruktur einstückig ausgebildet sind.

Dabei bedeutet "einstückig", dass das Stent-Gerüst und die Verankerungsstruktur aus dem gleichen Draht bzw. aus dem gleichen gelaserten Rohr gefertigt wurden.

Bei der erfindungsgemäßen Gefäßprothese sind am proximalen Ende des Stent-Gerüsts eine, zwei oder drei Verankerungsstrukturen vorgesehen.

Durch das Vorsehen von mehr als einer Verankerungsstruktur kann die Gefäßprothese noch sicherer im Herzen fixiert werden. Die zusätzlichen Verankerungsstrukturen weisen dabei vorzugsweise die gleiche Struktur und Form auf, oder aber unterschiedliche Formen; allen gemein ist in diesen Fällen aber die schlaufenförmige Grundstruktur.

Gemäß einer Weiterbildung der erfindungsgemäßen intraluminalen Gefäßprothese ist bevorzugt, wenn das Stent-Gerüst und/oder die drahtförmige Verankerungsstruktur an ihrer der Gefäßwand abgewandten und/oder zugewandten Seite Haken oder Dornen aufweisen.

Mit diesen Ausführungsformen wird die Fixierung der Gefäßprothese im Gefäß weiter verstärkt und gesichert. Die Haken und/oder Dornen können aus dem gleichen Material wie die Verankerungsstruktur bzw. das Stent-Gerüst sein, oder aber aus unterschiedlichem Material.

In der Ausführungsform der an der Innenseite der Gefäßprothese vorgesehenen Haken/Dornen ist von Vorteil, dass über diese Haken bzw. Dornen die Verankerung einer hierin zumindest teilweise einzuführenden Herzklappenprothese noch weiter unterstützt wird. Zwar wird bereits mit dem teilweisen Freisetzen und "Aufspannen" der Herzklappenprothese in dem proximalen Ende der erfindungsgemäßen Gefäßprothese eine Verankerung dieser in der Gefäßprothese gesichert, die Dornen und Haken können aber die Verankerung der Herzklappe in der Gefäßprothese zusätzlich unterstützen.

Gemäß einer weiteren Ausführungsform der erfindungsgemäßen intraluminalen Gefäßprothese ist bevorzugt, wenn das Stent-Gerüst und/oder die drahtförmige Verankerungsstruktur Röntgenmarker umfasst.

Durch das Vorsehen von Röntgenmarkern an der Verankerungsstruktur wird dem behandelnden Arzt die korrekte Platzierung der Verankerungsstruktur und damit auch der Gefäßprothese insgesamt erleichtert, da er damit die Platzierung der Gefäßprothese im Herzen unter Röntgenkontrolle präzise vornehmen kann.

Die erfindungsgemäße intraluminale Gefäßprothese weist insgesamt vorzugsweise einen Durchmesser von zwischen ca. 20 mm bis ca. 48 mm, vorzugsweise von zwischen 24 mm bis ca. 44 mm auf.

Gemäß einer Ausführungsform ist der Durchmesser der Gefäßprothese dabei über deren Gesamtlänge im Wesentlichen konstant.

In einer Weiterbildung der erfindungsgemäßen intraluminalen Gefäßprothese ist vorgesehen, wenn diese ferner eine Herzklappenprothese umfasst, wobei die Herzklappenprothese derart ausgebildet ist, dass sie mit ihrem distalen Ende am proximalen Ende der intraluminalen Gefäßprothese freisetzbar ist und über diese im Bereich der Herzklappen fixierbar ist.

Bei dieser Ausführungsform liegen entsprechend zwei Prothesen vor, die gemeinsam ein erfindungsgemäßes System zur Behandlung einer Aortendissektion, insbesondere vom Typ A, darstellen: Mit der erfindungsgemäßen Gefäßprothese wird, wie weiter oben erwähnt, eine Verankerungsstruktur bereitgestellt, die in einem ersten Schritt in das Herz fixiert wird und über welche eine Herzklappenprothese, die in einem zweiten Schritt eingeführt wird, sicher im Herzen verankert werden kann. Die Gefahr des Verrutschens oder einer Migration der Herzklappenprothese wird dadurch vermieden, und gelichzeitig ein relativ einfacher Aufbau des Systems erreicht.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung einer Ausführungsform einer erfindungsgemäßen Gefäßprothese, im nicht-eingeführten, expandierten Zustand in einer Ansicht der Längsseite;
- Fig. 2: eine schematische Darstellung der schrittweisen Freisetzung der Gefäßprothese aus Fig. 1 mit einem schematisch dargestellten Freisetzungskatheter; im vollständig geladenen Zustand (**A**); mit teilweise zurückgezogener Komprimierungsstruktur, aber mit immer noch am Freisetzungskatheter fixiertem proximalen und distalen Ende der Gefäßprothese (**B**); sowie mit freigesetztem proximalen Ende (**C**);
- Fig. 3: die schematische Darstellung einer im Herzgefäß vollständig freigesetzten Ausführungsform der erfindungsgemäßen Gefäßprothese;
- Fig. 4: die schematische Darstellung eines erfindungsgemäßen Systems, mit einer in einer erfindungsgemäßen Gefäßprothese verankerten Herzklappenprothese, wobei die Darstellung außerhalb des Herzgefäßes gezeigt ist.

In den Figuren werden gleiche Merkmale mit gleichen Bezugszeichen versehen, wobei aus Gründen der Übersichtlichkeit nicht in allen Figuren immer sämtliche Bezugszeichen angegeben sind.

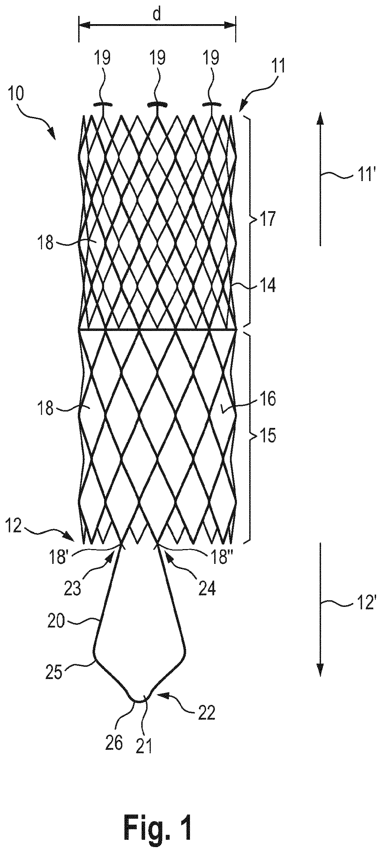

In den Figuren 1 bis 4 ist mit 10 insgesamt eine erfindungsgemäße Gefäßprothese bezeichnet, mit einem distalen Ende 11 und einem proximalen Ende 12. Die Gefäßprothese weist ein röhrchenförmiges Stent-Gerüst 14 auf, an welches in einem proximalen Abschnitt 15 ein Prothesen-Material 16 angebracht ist, wodurch der proximale Abschnitt 15 gecovert ist. Die Gefäßprothese 10 umfasst ferner einen distalen Abschnitt 17, der frei von Prothesen-Material ist.

Erfindungsgemäß kann das Stent-Gerüst 14 ein Laser-geschnittenes Stent-Gerüst sein, oder aber ein aus mehreren Drähten geflochtenes oder gewebtes Stent-Gerüst, oder aber aus einzelnen, mäanderförmig umlaufenden, hintereinander angeordneten Stent-Ringen bestehen, die nur über das Prothesen-Material miteinander verbunden sind.

Die Gefäßprothese 10 weist in dem in den Figuren gezeigten Beispiel rautenförmige Zellen 18 auf, die reihenförmig das röhrchenförmige bzw. zylinderförmige Stent-Gerüst 14 bilden. Am distalen Ende 11 sind Fixierstrukturen 19 an drei freien Ecken der rautenförmigen Zellen 18 am distalen Ende 11 vorgesehen, über welche das distale Ende 11 der Gefäßprothese 10 in einem Freisetzungskatheter (nicht gezeigt) in entsprechenden Aufnahmen bzw. Aussparungen aufgenommen und über eine hierüber geführte Rückzugshülle (nicht gezeigt) am Freisetzungskatheter fixiert werden können. Die Fixierstrukturen 19 sind in der in Fig. 1 gezeigten Ausführungsform T-förmig.

Am proximalen Ende 12 der Gefäßprothese 10 ist eine drahtförmige Verankerungsstruktur 20 vorgesehen, die in proximale Richtung 12' eine Schlaufe 21 bildet. Die Verankerungsstruktur 20 steht mit ca. 1/3 der Gesamtlänge der Gefäßprothese 10 über deren proximales Ende 12 hinaus.

Die Verankerungsstruktur 20 weist ein proximales Ende 22 auf, in dessen Bereich die Schlaufe 21 gebildet ist. Die Verankerungsstruktur 20 umfasst ferner zwei distale Enden 23 und 24, die an zwei freien Ecken 18', 18" der rautenförmigen Zellen 18 am proximalen Ende 12 der Gefäßprothese fixiert bzw. einstückig mit diesen ausgebildet sind.

Wie Fig. 1 zu entnehmen ist, sind die distalen Enden 23, 24 der Verankerungsstruktur 20 an unmittelbar benachbarten Ecken der rautenförmigen Zellen 18 am proximalen Ende 12 der Gefäßprothese 10 angebracht/einstückig mit diesen vorgesehen.

"Freie Enden" der rautenförmigen Zellen bedeuten dabei, wie auch für jede andere Ausführungsform der erfindungsgemäßen Gefäßprothese, das diese Ecken nicht mit einer weiteren rautenförmigen Zelle verbunden sind, sondern frei in die distale Richtung 11' oder proximale Richtung 12' ragen.

Fig. 1 ist ferner zu entnehmen, dass die Verankerungsstruktur 20 im Bereich der Schlaufe 21 eine trapezförmige Erweiterung 25 nach außen aufweist. Dies bedeutet, dass der Abstand der distalen Enden 23, 24 der Verankerungsstruktur 20 im Bereich deren Fixierung/Anbringung am proximalen Ende 12 der Gefäßprothese 10 kleiner ist als der Abstand der sich gegenüber liegenden Drähte der Schlaufe 21 im proximalen Bereich 22.

Die Verankerungsstruktur 20 weist, wie weiterhin in Fig. 1 gezeigt ist, in ihrer Schlaufe 21 ein äußerstes tropfenförmiges proximales Ende 26 auf.

Die Verankerungsstruktur 20 und das Stent-Gerüst 14 können einstückig gebildet sein, bspw. durch Laser-Schneiden eines entsprechenden Röhrchens bzw. Zylinders.

Die Verankerungsstruktur 20 und/oder das Stent-Gerüst können an ihrer jeweils der Gefäßwand abgewandten Seite Haken oder Dornen aufweisen (nicht gezeigt), sowie einen oder mehrere Röntgenmarker umfassen.

Der Durchmesser d der erfindungsgemäßen Gefäßprothese liegt vorzugsweise zwischen 20 mm und 48 mm, vorzugsweise zwischen ca. 24 mm und ca. 44 mm.

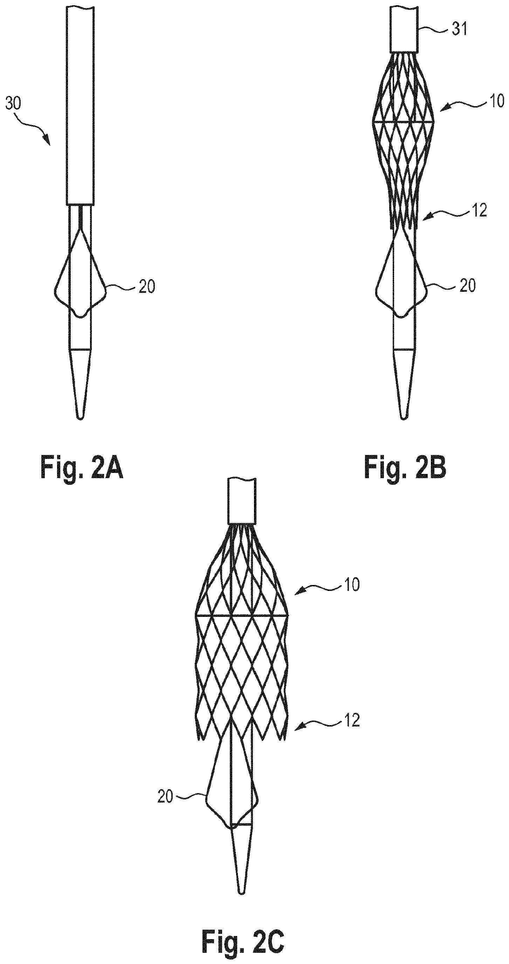

In Fig. 2 ist schematisch die Freisetzung einer auf einen Freisetzungskatheter 30 geladenen erfindungsgemäßen Gefäßprothese10 gezeigt. Die Gefäßprothese 10 ist dabei an Ihrem distalen Ende 11 über die dort vorgesehenen Fixierstrukturen 19 am Freisetzungskatheter in entsprechenden Aufnahmen und durch Überziehen einer Rückzugshülle 31 fixiert. Die Gefäßprothese 10 ist auch über ihr proximales Ende 12 am Freisetzungskatheter 30 über ein entsprechendes System (nicht gezeigt) lösbar fixiert. Der vollständig fixierte und komprimierte Zustand der Gefäßprothese 10 ist in Fig. 2A gezeigt.

Nach Zurückziehen der die Gefäßprothese komprimiert haltenden Rückzugshülle 31 expandiert sich ein mittlerer Bereich der Gefäßprothese 10 ballonförmig, während das proximale und das distale Ende 12, 11 der Gefäßprothese 10 noch am Freisetzungskatheter 30 fixiert sind (siehe Fig. 2B).

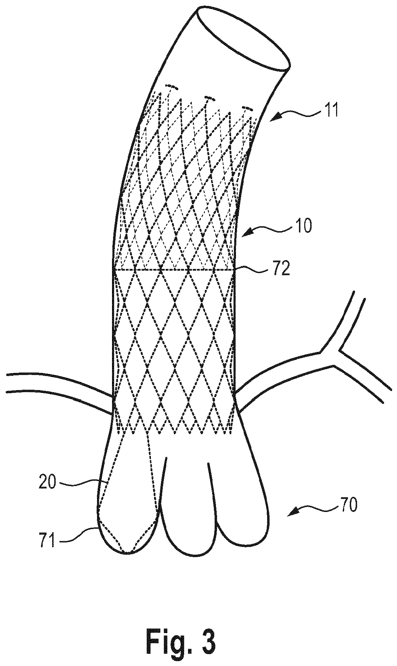

Nach der Freisetzung des proximalen Endes 12 der Gefäßprothese 10 kann dieses expandieren, wohingegen das distale Ende 11 der Gefäßprothese 10 noch am Freisetzungskatheter 30 und durch die Rückzugshülle 31 fixiert und komprimiert ist (siehe Fig. 2C). In diesem Zustand kann die erfindungsgemäße Gefäßprothese 10 an dem gewünschten Ort freigesetzt und positioniert werden, und zwar vorzugsweise hinter einem Segel der Aortenklappe (siehe hierzu auch Fig. 3), vorzugsweise hinter das nonkoronare Segel bzw. "non coronary cusp "NCC", positioniert. Dadurch kann die Verankerungsstruktur 20 genau ausgerichtet werden und eine spätere proximale Migration der Gefäßprothese verhindern.

Ist die Positionierung der Verankerungsstruktur 20 gesichert, kann die restliche Gefäßprothese durch vollständiges Rückziehen der Rückzugshülle 31 freigesetzt werden. Der in einem Herzen 70 vollständig freigesetzte Zustand der Gefäßprothese 10 ist in Fig. 3 gezeigt, in welcher Figur die Gefäßprothese der besseren Übersicht halber gestrichelt gezeigt ist. Zu erkennen ist hier, dass Verankerungsstruktur 20 hinter einem Segel der Aortenklappe 71 positioniert ist während sich der Rest der Gefäßprothese 10 nach distal in die aufsteigende Aorta 72 erstreckt.

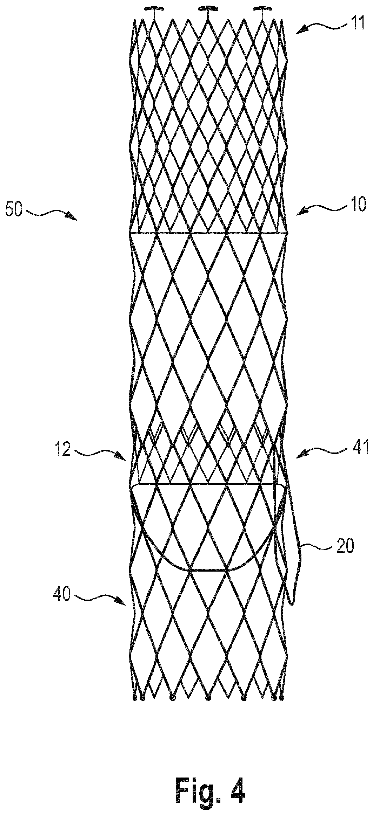

Fig. 4 zeigt schließlich ein erfindungsgemäßes System 50 aus einer Gefäßprothese 10 und einer Herzklappenprothese 40 in zusammengesetzter Form, und zwar außerhalb des Herzens der besseren Übersicht wegen.

Sobald die Gefäßprothese 10 als Anker im Herzen positioniert ist, kann die Herzklappenprothese 40 über diese eingeführt und derart freigesetzt werden, dass sich die Herzklappenprothese 40 mit ihrem distalen Ende 41 in der Gefäßprothese 10 zumindest teilweise an deren proximalen Ende 12 verhakt bzw. bereits durch Expansion dort fixiert.

## Patentansprüche

1. Intraluminale Gefäßprothese (10) zur Implantation in das Herz (70) und/oder in Herzgefäße eines Patienten, insbesondere zur Verankerung im Bereich der Herzklappensegel (71), mit einem distalen (11) und einem proximalen (12) Ende, wobei das distale Ende (11) in Bezug auf den Blutstrom weiter stromabwärts liegt und das proximale Ende (12) in Bezug auf den Blutstrom weiter stromaufwärts liegt, wobei die Gefäßprothese (10) ein Stent-Gerüst (14) aufweist, sowie ein Prothesen-Material (16), mit welchem das Stent-Gerüst (14) zumindest teilweise bedeckt ist, derart, dass ein gecoverter proximaler Abschnitt (15) und ein nicht-gecoverter distaler Abschnitt (17) gebildet wird, **dadurch gekennzeichnet, dass** die Gefäßprothese (10) am proximalen Ende (12) zumindest eine bis drei längliche drahtförmige Verankerungsstruktur(en) (20) aufweist, welche im Wesentlichen schlaufenförmig ist/sind, und welche jeweils mit zwei Enden (23; 24) am Stent-Gerüst (14) fixiert ist/sind und welche jeweils mit ihrem proximalen Ende (22) in Form einer Schlaufe (21) in proximale Richtung (12') weisend und über das proximale Ende (12) des Stent-Gerüsts (14) hinaus ragend positioniert ist/sind, und wobei die drahtförmige Verankerungsstruktur (20) im Bereich der Schlaufe (21) eine trapezförmige Erweiterung (25) umfasst.

2. Intraluminale Gefäßprothese (10) nach Patentanspruch 1, **dadurch gekennzeichnet, dass** die distalen Enden (23; 24) der drahtförmigen Verankerungsstruktur (20) an unmittelbar benachbarten Ecken (18'; 18") zweier rautenförmigen Zellen (18) positioniert sind.

3. Intraluminale Gefäßprothese (10) nach einem der vorstehenden Patentansprüche, **dadurch gekennzeichnet, dass** das äußerste proximale Ende (26) der drahtförmigen Verankerungsstruktur tropfenförmig ist.

4. Intraluminale Gefäßprothese (10) nach einem der vorstehenden Patentansprüchen, **dadurch gekennzeichnet, dass** das Stent-Gerüst (14) ein laser-geschnittenes Stent-Gerüst oder ein geflochtenes oder gewebtes Stent-Gerüst ist.

5. Intraluminale Gefäßprothese (10) nach einem der vorstehenden Patentansprüchen, **dadurch gekennzeichnet, dass** das Stent-Gerüst (14) und die Verankerungsstruktur (20) einstückig ausgebildet sind.

6. Intraluminale Gefäßprothese (10) nach einem der vorstehenden Patentansprüche, **dadurch gekennzeichnet, dass** das Stent-Gerüst (14) und/oder die drahtförmige Verankerungsstruktur (20) an ihrer der Gefäßwand abgewandten Seite Haken oder Dornen aufweisen.

7. Intraluminale Gefäßprothese (10) nach einem der vorstehenden Patentansprüche, **dadurch gekennzeichnet, dass** das Stent-Gerüst (14) an seiner der Gefäßwand zugewandten Seite Haken oder Dornen aufweist.

8. Intraluminale Gefäßprothese (10) nach einem der vorstehenden Patentansprüchen, **dadurch gekennzeichnet, dass** das Stent-Gerüst (14) und/oder die drahtförmige Verankerungsstruktur Röntgenmarker umfasst.

9. Intraluminale Gefäßprothese (10) nach einem der vorstehenden Patentansprüchen, **dadurch gekennzeichnet, dass** sie einen Durchmesser von zwischen ca. 20 mm bis ca. 48 mm, vorzugsweise von zwischen 24 mm bis ca.44 mm aufweist.

10. Intraluminale Gefäßprothese (10) nach einem der Ansprüche 1 bis 9, ferner umfassend eine Herzklappenprothese (40), wobei die Herzklappenprothese (40) derart ausgebildet ist, dass sie mit ihrem distalen Ende (41) am proximalen Ende (12) der intraluminalen Gefäßprothese (10) freisetzbar ist und über diese im Bereich der Herzklappen fixierbar ist.

## Claims

1. Intraluminal vascular prosthesis (10) for implantation into the heart (70) and/or into cardiovascular vessels of a patient, in particular for anchoring in the region of the cardiac valve leaflets (71), having a distal (11) and a proximal (12) end, wherein the distal end (11) lies further downstream in relation to the blood flow and the proximal end (12) lies further upstream in relation to the blood flow, the vascular prosthesis (10) having a stent support (14), and a prosthesis material (16) with which the stent support (14) is at least partially covered, such that a covered proximal portion (15) and a non-covered distal portion (17) are formed, **characterized in that** the vascular prosthesis (10) comprises, at the proximal end (12), at least one to three elongate wire-shaped anchoring structure(s) (20) which is/are substantially loop-shaped and which via two ends (23; 24) is/are fixed to the stent support (14) and which is/are positioned with its proximal end (22) in the form of a loop (21) pointing towards the proximal direction (12') and projecting beyond the proximal end (12) of the stent support (14), and wherein the wire-shaped anchoring structure (20) comprises a trapezoidal extension (25) in the region of the loop (21).

2. The intraluminal vascular prosthesis (10) according to claim 1, **characterized in that** the distal ends (23; 24) of the wire-shaped anchoring structure (20) are positioned at immediately adjacent corners (18'; 18") of two diamond-shaped cells (18).

3. The intraluminal vascular prosthesis (10) according to any of the preceding claims, **characterized in that** the outermost proximal end (26) of the wire-shaped anchoring structure is drop-shaped.

4. The intraluminal vascular prosthesis (10) according to any of the preceding claims, **characterized in that** the stent support (14) is a laser-cut stent support or a braided or woven stent support.

5. The intraluminal vascular prosthesis (10) according to any of the preceding claims, **characterized in that** the stent support (14) and the anchoring structure (20) are integrally formed.

6. The intraluminal vascular prosthesis (10) according to any of the preceding claims, **characterized in that** the stent support (14) and/or the wire-shaped anchoring structure (20) have hooks or spikes on their respective side facing away from the vascular wall.

7. The intraluminal vascular prosthesis (10) according to any of the preceding claims, **characterized in that** the stent support (14) has hooks or spikes on its side facing the vascular wall.

8. The intraluminal vascular prosthesis (10) according to any of the preceding claims, **characterized in that** the stent support (14) and/or the wire-shaped anchoring structure comprises X-ray markers.

9. The intraluminal vascular prosthesis (10) according to any of the preceding claims, **characterized in that** it has a diameter of between approx. 20 mm to approx. 48 mm, preferably of between 24 mm to approx. 44 mm.

10. The intraluminal vascular prosthesis (10) according to any of claims 1 to 9, further comprising a heart valve prosthesis (40), wherein the heart valve prosthesis (40) is designed such that it can be released with its distal end (41) at the proximal end (12) of the intraluminal vascular prosthesis (10) and can be fixed via the latter in the region of the heart valves.

## Revendications

1. Prothèse vasculaire intraluminale (10) destinée à être implantée dans le coeur (70) et/ou dans des vaisseaux coronaires d'un patient, en particulier pour l'ancrage dans la région des feuillets de valve cardiaque (71), comprenant une extrémité distale (11) et une extrémité proximale (12), l'extrémité distale (11) étant située plus en aval par rapport au flux sanguin et l'extrémité proximale (12) étant située plus en amont par rapport au flux sanguin, la prothèse vasculaire (10) présentant une armature d'endoprothèse (14) ainsi qu'un matériau de prothèse (16) avec lequel l'armature d'endoprothèse (14) est au moins en partie recouverte, de telle sorte qu'une portion proximale couverte (15) et une portion distale non couverte (17) soient formées, **caractérisée en ce que** la prothèse vasculaire (10) présente, à l'extrémité proximale (12), au moins une à trois structures d'ancrage allongées en forme de fil (20) qui sont essentiellement en forme de boucles et qui sont fixées à chaque fois par deux extrémités (23 ; 24) à l'armature d'endoprothèse (14), et qui sont positionnées à chaque fois avec leur extrémité proximale (22) en forme de boucle (21) orientée dans la direction proximale (12') et en saillie au-delà de l'extrémité proximale (12) de l'armature d'endoprothèse (14), et la structure d'ancrage en forme de fil (20) comprend, dans la région de la boucle (21), un élargissement trapézoïdal (25).

2. Prothèse vasculaire intraluminale (10) selon la revendication 1, **caractérisée en ce que** les extrémités distales (23 ; 24) de la structure d'ancrage en forme de fil (20) sont positionnées au niveau de coins directement adjacents (18' ; 18") de deux cellules en forme de losange (18).

3. Prothèse vasculaire intraluminale (10) selon l'une quelconque des revendications précédentes, **caractérisée en ce que** l'extrémité proximale la plus extérieure (26) de la structure d'ancrage en forme de fil est en forme de goutte.

4. Prothèse vasculaire intraluminale (10) selon l'une quelconque des revendications précédentes, **caractérisée en ce que** l'armature d'endoprothèse (14) est une armature d'endoprothèse découpée au laser ou une armature d'endoprothèse tressée ou tissée.

5. Prothèse vasculaire intraluminale (10) selon l'une quelconque des revendications précédentes, **caractérisée en ce que** l'armature d'endoprothèse (14) et la structure d'ancrage (20) sont réalisées d'une seule pièce.

6. Prothèse vasculaire intraluminale (10) selon l'une quelconque des revendications précédentes, **caractérisée en ce que** l'armature d'endoprothèse (14) et/ou la structure d'ancrage en forme de fil (20) présentent, au niveau de leur côté opposé à la paroi du vaisseau, des crochets ou des épines.

7. Prothèse vasculaire intraluminale (10) selon l'une quelconque des revendications précédentes, **caractérisée en ce que** l'armature d'endoprothèse (14) présente, au niveau de son côté tourné vers la paroi du vaisseau, des crochets ou des épines.

8. Prothèse vasculaire intraluminale (10) selon l'une quelconque des revendications précédentes, **caractérisée en ce que** l'armature d'endoprothèse (14) et/ou la structure d'ancrage en forme de fil comprennent des marqueurs radiographiques.

9. Prothèse vasculaire intraluminale (10) selon l'une quelconque des revendications précédentes, **caractérisée en ce qu'**elle présente un diamètre compris entre environ 20 mm et environ 48 mm, de préférence compris entre 24 mm et environ 44 mm.

10. Prothèse vasculaire intraluminale (10) selon l'une quelconque des revendications 1 à 9, comprenant en outre une prothèse de valve cardiaque (40), la prothèse de valve cardiaque (40) étant réalisée de telle sorte qu'elle puisse être libérée avec son extrémité distale (41) à l'extrémité proximale (12) de la prothèse vasculaire intraluminale (10) et qu'elle puisse être fixée par le biais de celle-ci dans la région des valves cardiaques.
